(19) Europäisches Patentamt / European Patent Office / Office européen des brevets

(11) **EP 4 461 215 A1**

(12) **EUROPEAN PATENT APPLICATION**
published in accordance with Art. 153(4) EPC

(43) Date of publication:
**13.11.2024 Bulletin 2024/46**

(21) Application number: **23737287.5**

(22) Date of filing: **04.01.2023**

(51) International Patent Classification (IPC):
**A61B 5/0537** (2021.01)     **G16H 20/00** (2018.01)

(52) Cooperative Patent Classification (CPC):
**A61B 5/0537; G16H 20/00**

(86) International application number:
**PCT/JP2023/000024**

(87) International publication number:
**WO 2023/132336 (13.07.2023 Gazette 2023/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2022 JP 2022000697**

(71) Applicant: **Tanita Corporation**
**Tokyo 174-8630 (JP)**

(72) Inventors:
• **KODAMA, Miyuki**
**Tokyo 174-8630 (JP)**

• **TANIDA, Senri**
**Tokyo 174-8630 (JP)**
• **KUBOTA, Tomomi**
**Tokyo 174-8630 (JP)**
• **SHIOTA, Takayuki**
**Tokyo 174-8630 (JP)**
• **NAGAHAMA, Toshiki**
**Tokyo 174-8630 (JP)**

(74) Representative: **Eisenführ Speiser**
**Patentanwälte Rechtsanwälte PartGmbB**
**Postfach 10 60 78**
**28060 Bremen (DE)**

(54) **BODY ASSESSMENT SYSTEM AND BODY ASSESSMENT PROGRAM**

(57)     A physical assessment system is provided that can set appropriate target values for the index of user's body and show the relationship between the index values of the user and the target values. The body assessment system (100) comprises: a body composition estimation section (54) that estimates body composition values for one or more items of the user based on the bioelectrical impedance of one or more body parts of the user; an index value acquisition section (56) that acquires the index value of the user based on one or more body composition values for an index to asses the body for a predetermined purpose; he target value acquisition section (57) that calculates target values for each user according to the physical characteristics of the user for each index; and a screen information generation section (59) that generates screen information showing the relationship between the index value and the target value.

FIG. 2

EP 4 461 215 A1

**Description**

[Cross-reference to related applications]

In this application, the benefits of patent application number 2022-000697, which was filed in Japan on January 5, 2022, are claimed, and the content of said application is hereby incorporated by reference.

[Field]

This technology relates to a body assessment system and body assessment program for assessing the body of a user.

[Background]

A body composition analyzer that measures the bioelectrical impedance of one or more body parts of a user and estimates the user's body composition based on the bioelectrical impedance is known (see, for example, Patent Publication No. JP2002-063278). In such a body composition analyzer, the body composition of multiple items such as body fat percentage, muscle mass, BMI, visceral fat level, basal metabolic rate, bone mass, and body water percentage is estimated and shown to the user.

[Summary]

This technology provides a new body assessment system that uses estimated body composition to provide useful information to users.

Simply showing the values of the multiple estimated body composition items does not help many users understand which body composition items need to be improved by how much in order to achieve their ideal body. Therefore, as a body assessment system that assesses the body based on body composition, a body assessment system is proposed that selects the body composition of the relevant items to obtain an ideal body, and presents the relationship between the target value of the relevant item and the body composition value of the relevant item obtained by the body composition analyzer to the user.

According to this body assessment system, the user can find out which body composition values for which items need to be improved to what extent in order to achieve the ideal body.

Here, the ideal body differs depending on the user's physical attributes such as gender, age, and race, so the target values also differ depending on gender, age, and race. Therefore, in the above body assessment system, ideal body composition values are stored for each gender, age, and/or race, and the ideal body composition value corresponding to the user's gender, age, and/or race is selected, and the relationship between the selected body composition value as the target value and the estimated body composition value is shown.

However, selecting the ideal body composition values based on the user's actual gender, age, and/or race may not always be appropriate. For example, when it comes to gender, the user is asked to select whether they are male or female, but there are some users whose physical characteristics are closer to the physical characteristics of the average female, even if their gender as a physical attribute is male, and vice versa. In this case, it is not appropriate to simply divide people into two categories (male or female) and set the target value as the ideal body composition values for men or women according to the actual gender, and it is preferable to set the target value according to the physical characteristics of the user in question. The same can be said for other physical attributes such as age and race.

Therefore, this technology provides a body assessment system that can set appropriate target values for the user's physical indices and show the relationship between the user's index values and those target values.

One embodiment of a body assessment system comprises: a body composition acquisition section that acquires body composition values for one or more items of a user, which are estimated based on the bioelectrical impedance of one or more body parts of the user; an index value acquisition section that acquires an index value of the user based on one or more body composition values for the index used to assess a body for a predetermined purpose; a target value acquisition section that calculates a target value for each of the users, according to the physical characteristics of the users, for the the index; and a screen information generation section that generates screen information showing a relationship between the index value and the target value.

With this configuration, the target value that is compared with the index value obtained from the user's body composition values is calculated for each user according to the user's physical characteristics, so it is possible to show the relationship between the user's index value by setting appropriate target value, rather than relying solely on the user's actual physical attributes. Furthermore, the body assessment system may consist of a body composition analyzer alone, or it may consist of a body composition analyzer and a communication-capable information processing device (e.g., a server device), or it may consist of a combination of a body composition analyzer and such an information processing device.

In the above body assessment system, the target value acquisition section may calculate the target value according to the predetermined purpose of the user.

This configuration allows the target value to be calculated according to the purpose of the user.

In the above body assessment system, the index value acquisition section may select one or more body composition items from the body composition values of the one or more items for assessing the body for the specified purpose, and acquire the body composition values of the selected items as the index values.

This configuration allows the relationship between the target value and the body composition values of the items corresponding to the purpose to be shown.

In the above body assessment system, the index value acquisition section may acquire the index value by inputting the body composition values of the one or more items into an inference model for calculating the index value.

This configuration allows the relationship between the index value and the target value for any index according to the purpose to be shown.

In the above body assessment system, the target value acquisition section may calculate the physical characteristics of the user based on the body composition values of one or more items acquired by the body composition acquisition section, and calculate the target value based on the calculated physical characteristics.

With this configuration, the user's physical characteristics for calculating the target value can be calculated from the body composition.

The above body assessment system may further comprise a default target value memory that stores default target values for each physical attribute for the index, and the target value acquisition section may calculate the target value based on the calculated physical characteristics and the default target values.

Using the default target values prepared for each physical attribute, this configuration allows the target value to be calculated without relying solely on the user's physical attributes.

In the above body assessment system, the default target value memory may store the target values according to the predetermined purpose.

With this configuration, the default target values can be used according to the user's purpose.

In the above body assessment system, the target value acquisition section may estimate the degree of the physical attributes of the user as the physical characteristics, and calculate the target value by weighting and adding the default target values for each of the physical attributes according to the degree of the physical attributes.

With this configuration, the degree of physical attributes is estimated as physical characteristics, so the target value can be calculated without relying solely on the user's actual physical attributes.

In the above body assessment system, the target value acquisition section may calculate the target value based on a type representing the physical characteristics of the user as determined from one or more predetermined perspectives.

This configuration allows the target value to be calculated based on the user's body type.

In the above body assessment system, the physical attribute may be gender, the default target value memory section may store default target values for males and females respectively, and the target value acquisition section may calculate the target value by estimating a gender degree of the user as the degree of the physical attribute of the user based on the body composition values of one or more items, and calculate the targe value by weighting and adding the default target values for males and females according to the estimated gender degree.

With this configuration, even for users whose actual gender is male but whose physical characteristics are closer to the physical characteristics of an average female, or vice versa, it is possible to set appropriate target values by modifying the default target values prepared for male or female users based on the gender degree (male and female degree) of the user, rather than the default target values prepared for male or female users.

In the above body assessment system, the physical attribute may be age, the default target value acquisition section may store the default target values for each generation according to the purpose, and the target value acquisition section may calculate the target value by estimating an internal age of the user as the degree of the physical attributes of the user based on the body composition values of one or more items, and calculate the target value by weighting and adding the default target values for each generation according to the estimated internal age.

With this structure, it is possible to set appropriate target values by modifying the default target values prepared for each generation according to the estimated internal age, rather than the actual age.

In the above body assessment system, the physical attribute may be race, the default target value acquisition section may store the default target values for each race, and the target value acquisition section may calculate the target value by estimating a racial degree of the user as the degree of the physical attribute of the user based on the body composition values of the one or more items, and calculate the target value by weighting and adding the default target values for each race according to the estimated racial degree.

With this configuration, even for users whose actual race is Asian but whose physical characteristics are closer to those of the average Westerner, for example, it is possible to set appropriate target values by modifying the default target values prepared for each race based on the user's degree of race, rather than using the default target values prepared for each race.

In the above body assessment system, the target value acquisition section may calculate the target value as a target range defined by an upper limit and a lower limit.

This configuration allows the relationship between the index value and the target range to be shown.

In the above body assessment system, the purpose may be selectable from at least one of health, dieting, anti-aging, ideal athletes for a specific sport, and specific celebrities.

This configuration allows the relationship between the index value and the target value to be shown according to the various purpose.

The above body assessment system may further comprise: a bioelectrical impedance (BI) acquisition section that acquires the bioelectrical impedance of one or more body parts of the user by measuring the user's body; and a estimation section that estimates the body composition based on the bioelectrical impedance, wherein the body composition acquisition section may acquire the body composition value of the user estimated by the estimation section based on the bioelectrical impedance acquired by the BI acquisition section.

With this configuration, it is possible to measure the user's body and obtain body composition values.

One embodiment of a body assessment program is configured so that when executed by a computer, the computer functions as: a body composition acquisition section that acquires body composition values for one or more items of a user, which are estimated based on the bioelectrical impedance of one or more body parts of the user; an index value acquisition section that acquires an index value of the user based on one or more body composition values for the index used to assess a body for a predetermined purpose; a target value acquisition section that calculates a target value for each of the users, according to the physical characteristics of the users, for the the index; and a screen information generation section that generates screen information showing a relationship between the index value and the target value.

With this configuration, the target value that is compared with the index value obtained from the user's body composition values is calculated for each user according to the user's physical characteristics, so it is possible to show the relationship between the user's index value by setting appropriate target value, rather than relying solely on the user's actual physical attributes.

[Brief Explanation of the Drawing]

Figure 1 shows an external configuration of a body assessment system in an embodiment of this technology;

Figure 2 is a block diagram showing a configuration of the body assessment system in an embodiment of this technology;

Figure 3 is a graph showing the difference between men and women in the relationship between body fat percentage and fasting plasma insulin levels;

Figure 4 shows an example of modifying a target value for body fat percentage in an embodiment of this technology;

Figure 5 shows an example of modifying the target values for basal metabolism and visceral fat levels in an embodiment of this technology;

Figure 6 shows an example of modifying the target values for muscle mass and body fat percentage in an embodiment of this technology;

Figure 7 shows an example of the target values for muscle mass and body fat percentage in an embodiment of this technology;

Figure 8 shows an example of an assessment result screen in an embodiment of this technology; and

Figure 9 shows an example of an assessment result screen in an embodiment of this technology.

[Embodiment]

The following is an explanation of the embodiment of this technology, with reference to the drawings. The embodiment described below is an example of how this technology can be implemented, and it is not intended to limit this technology to the specific configuration described below. When implementing this technology, the specific configuration corresponding to the embodiment may be adopted as appropriate.

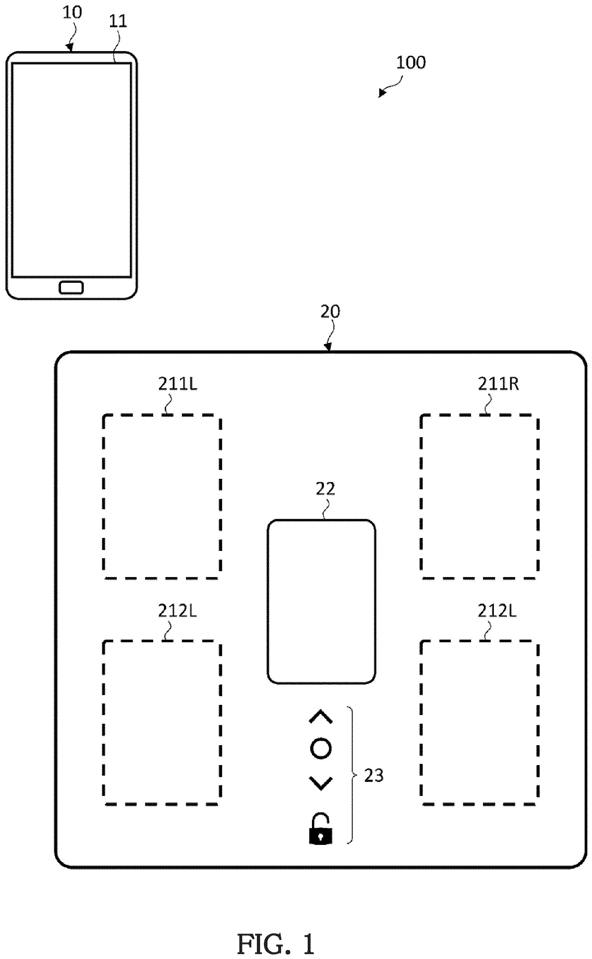

Figure 1 shows an external configuration of a body assessment system in an embodiment of this technology. As shown in Figure 1, the body assessment system 100 consists of an information processing terminal 10 and a body composition analyzer 20. The information processing terminal 10 is equipped with a touch panel 11 that serves as both an input device and a display. The body composition analyzer 20 is a device with a plate-like shape. The body composition scale 20 has a display panel 22 in the center of the top surface, and multiple operation buttons 23 below it. The body composition scale 20 measures the user's weight and estimates their body composition by having the user stand on it.

The information processing terminal 10 is a portable terminal equipped with a processor capable of executing application programs, memory temporarily storing data necessary for the processor's processing, internal storage such as flash memory storing application programs and generated data, etc., a touch panel, various interfaces, etc. In addition, the information processing terminal 10 is equipped with a wireless communication device for connecting to the Internet and a short-range communication device for connecting to other nearby devices. The body composition analyzer 20 is equipped with a short-range communication device for connecting to other nearby devices. The body composition analyzer 20 and the information processing terminal 10 can send and receive various types of information via short-range wireless communication (e.g., Bluetooth) by pairing with each other.

Instructions from the user to the body composition scale 20 can be input from the information processing terminal 10. In addition, the body weight measured by the body composition scale 20 and the estimated body composition are displayed on the display panel 22 and also displayed on the information processing terminal 10. For this purpose, an application is installed on the information processing terminal 10 to communicate with the body composition scale 20 and achieve the above functions. The information processing terminal 10 may be a general-purpose device such as a smartphone, or it may be a dedicated device used exclusively for the body composition scale 20.

The top surface of the body composition analyzer 20 is equipped with an electrode 211R for conducting electricity to the right foot in the upper right corner, an electrode 212R for measurement in the lower right corner, an electrode 211L for conducting electricity to the left foot in the upper left corner, and an electrode 212L for measurement in the lower left corner. By standing barefoot on the body composition analyzer 20, the user can measure their weight and estimate their body composition. At this time, the base of the right foot touches the current electrode 211R, the heel of the right foot touches the measurement electrode 212R, the base of the left foot touches the current electrode 211L, and the heel of the left foot touches the measurement electrode 212L.

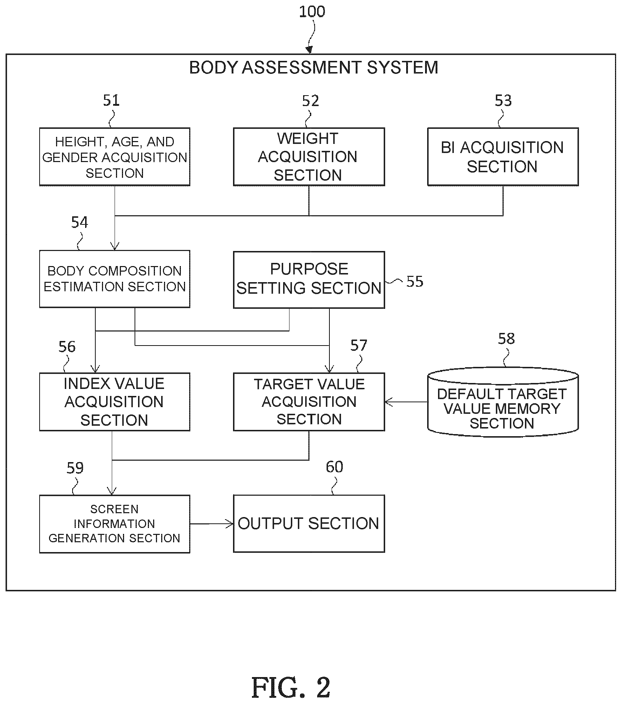

Figure 2 is a block diagram showing a configuration of the body assessment system in an embodiment of this technology. As shown in Figure 2, the body assessment system 100 is equipped with a height, age, and gender acquisition section 51, a weight acquisition section 52, a bioelectrical impedance (BI) acquisition section 53, and a body composition estimation section 54.

The height, age, and gender acquisition section 51 acquires the user's height, age, and gender information, which are the user's biological information, by receiving the user's operation input via the touch panel 11, which serves as the input section. In addition, the height, age, and gender acquisition section 51 may acquire the height, age, and gender information by receiving the user's operation input via the operation button 23. The height, age, and gender acquisition section 51 may be provided in either the information processing terminal 10 or the body composition analyzer 20.

The body weight acquisition section 52 acquires the user's body weight, which is the user's biometric information, by measuring the user's body weight. The body composition scale 20 has a load cell inside it for measuring body weight as the body weight acquisition section 52. The load cell is composed of a strain body, which is a metal member that deforms according to the load, and a strain gauge that is attached to the strain body. When the user steps on the body composition analyzer 20, the strain gauges expand and contract as the strain body of the load cell bends under the user's weight. The resistance value (output value) of the strain gauges changes in accordance with this expansion and contraction. The body composition analyzer 20 calculates the user's weight by comparing the output value (zero point) of the load cell when no load is applied to it with the output value when a load is applied to it. The structure for measuring body weight using a load cell is the same as that of a conventional bathroom scale.

The bioelectrical impedance of the user's entire body and each body part (hereafter, the entire body and each body part are referred to as "body parts") is measured by the BI acquisition section 53. The BI acquisition section 53 is equipped with left and right current electrodes 211R and 211L, and left and right measurement electrodes 212R and 212L. The left and right current electrodes 211R and 211L apply a weak electric current to the user's body. The left and right measurement electrodes 212R and 212L detect the potential difference (voltage) that occurs between them. The bioimpedance acquisition section 53 measures the bioelectrical impedance of the user when they are standing with both of their bare feet in contact with both the left and right current electrodes 211R and 211L and the left and right measurement electrodes 212R and 212L.

Specifically, the BI acquisition section 53 applies a weak alternating current to the left and right current electrodes 211R and 211L, and detects voltage (potential difference), and based on these, it determines the user's bioelectrical impedance (impedance Z, as well as the resistance component R and reactance component X of impedance Z). In addition, the BI acquisition section 53 uses the applied current and detected voltage to perform waveform processing such as discrete Fourier transform (DFT) processing to obtain the resistance component R and reactance component X of impedance Z.

In this way, the BI acquisition section 53 sends an alternating current constant current from each current electrode to a predetermined part of the user's body, and measures the potential difference that occurs in this current path. Then, based on these current and potential difference values, the bioelectrical impedance of the user's multiple body parts is calculated. The structure for measuring bioelectrical impedance can be the same as that of a general body composition analyzer. In addition, the bioelectrical impedance of each of the multiple body parts is determined when a constant current of a reference frequency (e.g., 50 kHz) is applied, when a constant current of a high frequency (e.g., 250 kHz) is applied, and when a constant current of a low frequency (e.g., 5 kHz) is applied.

The body composition estimation section 54 obtains the height, age, and gender obtained by the height, age, and gender acquisition section 51, the weight obtained by the weight acquisition section 52, and the bioelectrical impedance obtained by the BI acquisition section 53, and estimates the body composition value by performing calculations using this information. The body composition estimation section 54 may be provided in either the information processing terminal 10 or the body composition analyzer 20. The body composition estimation section 54 obtains measured body composition values for multiple items such as fat percentage, fat mass, lean mass, muscle mass, visceral fat mass, visceral fat level, visceral fat area, subcutaneous fat volume, basal metabolic rate, bone mass, body water percentage, BMI (Body Mass Index), intracellular fluid volume, extracellular fluid volume, etc. In addition, the value of an index that indicates muscle quality may be obtained as a measured body composition value. The structures for the body composition calculation can also use the same structures of a general body composition scale.

The height, age, and sex acquisition section 51, weight acquisition section 52, BMI acquisition section 53, and body composition estimation section 54 are structures that acquire body composition through measurement, and correspond to the body composition acquisition section of this technology. Furthermore, the body composition acquisition section of this technology may acquire body composition estimated by the body composition estimation section 54 provided in another device.

The body assessment system 100 is further provided with a purpose setting section 55, an index value acquisition section 56, a target value acquisition section 57, a default target value memory section 58, a screen information generation section 59, and an output section 60. The body assessment system 100 has a structure that obtains the user's index value based on the user's body composition value for the index used to assess the body for a predetermined purpose, calculates the target value, which is the ideal value for that index value, and shows the relationship between the user's index value and target value.

The purpose setting section 55 sets the purpose of what kind of body is considered to be an ideal body. The possible purposes that can be set are: becoming healthy, dieting, anti-aging, avoiding metabolic syndrome, aiming for the body of an ideal athlete in a specific sport, or aiming for the body of a specific celebrity. The purpose setting section 55 sets the purpose selected by receiving the user's operation input via the touch panel 11 as the input section. The purpose setting section 55 may also set the purpose selected by receiving the user's operation input via the operation button 23. In addition, the purpose setting section 55 may set all of the one or more prepared purpose, respectively, without the user's selection.

The index value acquisition section 56 identifies the index for assessing the body based on the purpose set by the purpose setting section 55, and acquires the user's index value for that index. The index value acquisition section 56 acquires the index value based on one or more of the user's body composition values. Specifically, the index value acquisition section 56 selects one or more body composition items from the body composition values of one or more items estimated by the body composition estimation section 54 to assess the body for the set purpose, and acquires the body composition values of the selected items as the index values, or acquires the index values by inputting the body composition values of one or more items into the inference model for calculating the index values.

For example, if the purpose is to avoid metabolic syndrome, the index value acquisition section 56 selects visceral fat percentage from the user's body composition values as an index that has a significant correlation with the risk of metabolic syndrome, and acquires this as the index value for the user.

For example, if the purpose is to achieve the body of an ideal athlete for a particular sport, the index value acquisition section 56 selects muscle mass and body fat percentage from the user's body composition values as the index values for that user.

For example, if the purpose is to become healthier, the index value acquisition section 56 uses the health level calculated by a predetermined inference model as an index, and acquires the health level by inputting the user's multiple biometric information and body composition values into the inference model, and acquires this as the index value for the relevant user. The following explains the method for calculating the health level.

The index value acquisition section 56 uses a multiple regression equation obtained by performing statistical analysis such as multiple regression analysis on a large number of pairs of biological information and body composition values and health checkup results as an inference model to estimate health checkup results from biological information and body composition values. This multiple regression equation uses biological information and body composition values as explanatory variables and health checkup results as the objective variable. In addition, this multiple regression equation can also be considered an inference model that has been learned using a large number of pairs of biological information and body composition values and health checkup results obtained by actually conducting health checkups as the training data. Furthermore, the inference model is not limited to a multiple regression equation, and can also be other inference models that are generated through learning, such as decision trees or neural networks. For example, instead of using age and gender as explanatory variables, it may be possible to use different multiple regression equations for each age and gender.

For example, when fat mass (kg) is set as $x_1$ and visceral fat mass ($cm^2$) is set as $x_2$, the index value acquisition section 56 estimates HDL cholesterol Y using the following multiple regression formula (1) through multiple regression analysis. In addition, in order to improve the accuracy of the estimation of biochemical test values, for example, the change in and/or direction of change in $x_1$ and $x_2$ may be taken into account, and HDL cholesterol Y may be estimated using a multiple regression equation that also adds these as explanatory variables.

$$Y = a\ x_1\ /\ x_2^2 + b\ \ \ ...(1)$$

The index value acquisition section 56 stores a plurality of multiple regression equations such as the above for estimating multiple items of biochemical test values. The biological information and body composition values used to estimate each biochemical test value are generally different. The index value acquisition section 56 estimates each biochemical test value by substituting the necessary biological information and body composition values into the regression equation used to estimate each biochemical test value.

The index value acquisition section 56 determines the level of health based on the estimated multiple items of health checkup results. However, in this embodiment, not all items of results obtained from the actual health checkup are estimated as health checkup results, but in particular, biochemical test values are estimated. Therefore, the index value acquisition section 56 determines the level of health based on this information.

The index value acquisition section 56 may determine the overall prevalence risk of lifestyle-related diseases (hereinafter simply referred to as "prevalence risk"), which indicates the overall risk of lifestyle-related diseases, as the level of health. According to conventional methods, the prevalence risk is determined by, for example, calculating a comprehensive score based on chest X-rays, urine protein, occult blood in the urine, AST (GOT), $\gamma$-GT ($\gamma$-GTP), LDL cholesterol, HDL cholesterol, triglycerides, uric acid, HbA1c, FPG, hematocrit, red blood cells, CRP, systolic blood pressure, diastolic blood pressure, etc., but in this embodiment, the index value acquisition section 56 obtains the prevalent risk based on, for example, AST (GOT), $\gamma$-GT ($\gamma$-GTP), LDL cholesterol, HDL cholesterol, triglycerides, uric acid, HbA1c, hematocrit, and CRP, which are obtained by estimation in the index value acquisition section 56.

The index value acquisition section 56 may calculate the prevalent risk as the health level using a general score calculation formula that follows conventional general scoring methods and makes modifications to health checkup results (e.g., chest X-rays, blood pressure, etc.) for which no estimation results are obtained by the index value acquisition section 56. These modifications may simply omit items for which no estimation results have been obtained in the conventional total score calculation method.

Here, because the health checkup results estimated from biometric information and body composition values may be less accurate than the health checkup results obtained by actually conducting a health checkup, depending on the item, when the prevalent risk is obtained from the estimated health checkup results using the conventional method of calculating the total score, there may be cases where the value of the prevalent risk deviates from the actual prevalent risk.

In order to prevent such cases, there is a method of determining the health level from the estimated health checkup results using a multiple regression equation obtained by performing a statistical analysis such as multiple regression analysis on a large number of pairs of estimated health checkup results and health levels from a large number of pairs of biological information and body composition values. This multiple regression equation uses the health checkup results estimated from the biological information and body composition values as explanatory variables and the health level as the objective variable. This multiple regression equation can also be considered to be an inference model obtained by learning from a set of pairs of health checkup results estimated from a large number of sets of biological information and body composition values and the health level of the subjects from whom the biological information and body composition values were obtained.

In addition, the inference model is not limited to multiple regression equations, and can be other inference models generated by learning, such as decision trees or neural networks. For example, age and gender can be used without using them as explanatory variables, by preparing multiple regression equations that differ for each age and gender. In addition, since the body assessment system 100 obtains not only the estimated health checkup results but also the biological information and body composition values, it may be possible to make the biological information and body composition values explanatory variables as well as the estimated health checkup results.

Among the teacher data used in this training, the health level may be (1) the value of the prevalent risk calculated by conventional total scoring from the health checkup results (including information such as chest X-rays) obtained from the actual health checkup, may be used, or (2) information on whether or not the person actually has a lifestyle-related disease may be used, or (3) actual medical expenses may be used, considering the medical expenses incurred as an indication of whether or not the person has a lifestyle-related disease and the severity of the disease.

Regardless of whether you choose (1) to (3), the multiple regression equation that inputs the estimated health checkup results and outputs the health level is a different equation from the calculation formula used for the conventional total score. The formula calculates an index that combines the items in the health checkup results based on the properties of bioelectrical impedance measured using a different method to the conventional method, so that the relationship between the estimated health checkup results and health level becomes stronger through the above learning. The fact that it is possible to adjust the formula for determining health level by using the health checkup results means that even if the accuracy of the estimates from the body composition values for each of the multiple items in the health checkup results is low, it is possible to select a combination of multiple items that will have a high level of accuracy (degree of agreement with actual health risks from a specific perspective) in a limited situations, and this will make it possible to more reliably determine a user's health level by measuring bioelectrical impedance.

The target value acquisition section 57 calculates target values for each user according to the physical characteristics of that user for the indicators specified according to the purposes set in the purpose setting section 55. Specifically, the target value acquisition section 57 calculates the physical characteristics of the user based on the body composition values of one or more items estimated by the body composition estimation section 54, and calculates the target value based on the calculated physical characteristics.

The default target value memory section 58 stores the default target values for each physical attribute for the indices. For example, the default target value memory section 58 stores the ideal BMI range for each combination of generation and gender as the default target value for the BMI as the index for the purpose of dieting. In addition, the default target value memory section 58 stores the ideal muscle mass and body fat percentage combinations for each gender as the indices for the purpose of aiming for the body of a professional tennis player. In this way, the default target value memory section 58 may store default target values for each combination of multiple physical attributes. In addition, the default target value memory section 58 may store a combination of ideal values for multiple body composition items as default target values for a single purpose.

As described above, it is usually the case that the corresponding default target value is read from the default target value memory section 58 according to the user's physical attributes (gender, age, race, etc.) and used as the target value. However, there are cases where it is not appropriate to use the default target value prepared for men as it is, even if the user's actual physical attribute is male, but their physical characteristics are closer to the average female.

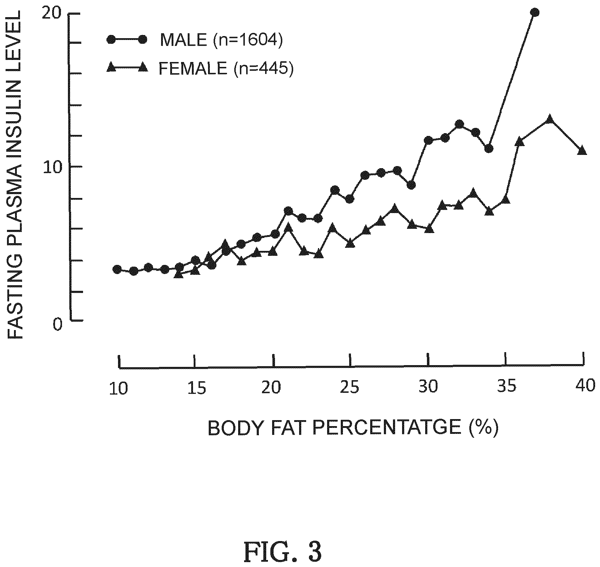

Figure 3 is a graph showing the difference between men and women in the relationship between body fat percentage and fasting plasma insulin levels. It is known that the higher the body fat percentage, the higher the fasting plasma insulin levels, which can cause disturbances in glucose metabolism and increase the risk of diabetes, as shown in Figure 3. However, as shown in Figure 3, the curve showing the increase in fasting plasma insulin levels relative to body fat percentage differs between men and women.

Therefore, the user's body fat percentage alone is not sufficient to determine whether the fasting plasma insulin level, i.e., the risk of diabetes, should be assessed as high or low. By selecting one of the two curves according to the user's gender, it is possible to assess the risk of diabetes from the body fat percentage. However, it may not always be appropriate to determine whether to use the male or female increase curve for a given user based on their gender as a physical attribute.

In other words, there is a range of variation in both the male and female curves, and there are men with high levels of body water and low levels of damage to the blood vessels, who have a lower risk even with the same body fat percentage, and there are also women with low levels of body water and high levels of cholesterol in the blood vessels, who are closer to the male curve. It is more effective to indicate the user's unique disease risk and healthy zone based on the user's unique body composition.

Therefore, the target value acquisition section 57 estimates the user's physical characteristics based on the body composition values of one or more items estimated by the body composition estimation section 54, and calculates target values suitable for the user based on the estimated physical characteristics. At this time, the target value acquisition section 57 calculates the target value based on the estimated physical characteristics and the default target values stored in the default target value memory section 58. In other words, in this embodiment, rather than making a uniform assessment or evaluation based on physical attributes such as gender or race, the target values for the relevant user are calculated based on factors such as muscle quality based on electrical resistance, body water balance, and fat distribution.

The target value acquisition section 57 estimates the degree of the physical attributes (type) of the relevant user in the assessment items for assessing the user from a certain aspect, based on the body composition values estimated by the body composition estimation section 54 for the user. The degree of physical attributes refers to the corresponding percentage of each individual user for the multiple types of physical attributes prepared for each assessment item, and includes, for example, the corresponding percentage of each individual user for the physical attributes of male and female prepared for the assessment item of gender (gender degree), and the corresponding percentage of each individual user for the physical attributes of Asian, Western, African, Hispanic, and other prepared for the assessment item of race. For example, the target value acquisition section 57 calculates the user's gender degree, i.e. the degree of male and female, by dividing the percentage for gender, and for age, it estimates the internal age, and for race, it calculates the degree of each race (Asian, Western, African, Hispanic, and other) by dividing the percentage. Specifically, the target value acquisition section 57 estimates the degree of gender, for example, as 60% male and 40% female. In addition, the target value acquisition section 57 may determine the degree of the user's physical attributes in the assessment items using the bioelectrical impedance measured by the BI acquisition section 53.

Here, the assessment items are one or more items used to assess the user from a certain aspect, such as gender, age, race/ethnicity, and evolution. The degree of an attribute (type) is calculated according to the bioelectrical impedance for a certain assessment item. For example, in the gender determination item, attributes such as male and female are set to determine the degree of the user's gender, while in the age determination item, attributes such as young, middle-aged, and elderly are set to determine the degree of the user's age, and in the race/ethnicity determination item, attributes such as Asian, Nordic, African-American, and Arab are set to determine the degree of the user's race, and in the evolution category, attributes such as ape-man, primitive man, archaic man, modern man, and contemporary man are set to determine the degree of the user's evolutionary stage, and the degree of each attribute is calculated.

In other words, since the characteristics of bioelectrical impedance when a weak electric current is passed through the body vary depending on physical characteristics such as the length of the limbs, cross-sectional area of the muscles, water content, and muscle fiber density, if the degree of an attribute can be calculated, it is possible to determine the degree of the attribute related to the assessment item of the user using the estimated body composition value or measured bioelectrical impedance, regardless of the user's actual age, gender, height, weight, etc.

Therefore, the target value acquisition section 57 calculates the degree of the attribute based on the user's estimated body composition value or measured bioelectrical impedance using various physical characteristic discrimination equations created in advance.

As discriminant equations for the degree of attributes, various body characteristic discriminant equations can be used, such as discriminant equations for characteristics based on body composition values for specific multiple items, characteristics based on the ratio of the length of slender parts to the length of the torso, characteristics based on the balance of the cross-sectional area of the muscles in the limbs, characteristics based on the density of muscle fibers, characteristics of the water content of the fat layer, and characteristics of the distribution of water in the muscles.

As assessment items, there are examples such as assessment items according to generation, assessment items according to gender, assessment items according to evolution, and assessment items according to race/ethnicity, as mentioned above, but these are not limited to these. The target value acquisition section 57 determines the degree of the attributes of the user's physical characteristics from the user's body composition values or bioelectrical impedance. The degree of the attribute to be determined is not necessarily limited to cases where it matches the actual physical characteristics, including biological sex and race, but may also be cases where it does not match the actual physical characteristics. For example, there may be cases where the degree of female is judged to be higher than the degree of male in the gender assessment item, even though the user is actually male. The target value acquisition section 57 can obtain the target value corresponding to the physical characteristics of the user by calculating the degree of the attribute corresponding to the physical characteristics of the user, without using objective facts that are obvious, such as being male, being in one's 20s, or being Asian.

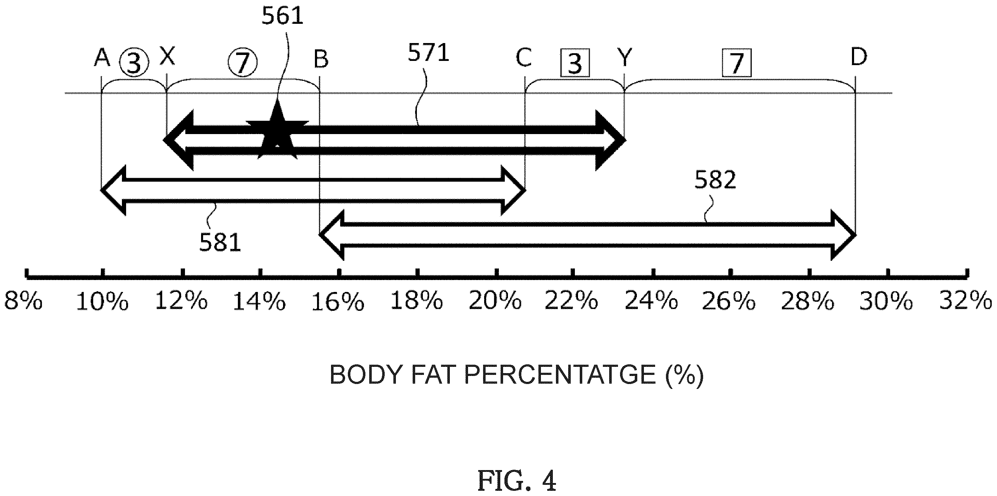

Figure 4 shows an example of modifying a target value for body fat percentage in an embodiment of this technology. The default target value memory section 58 stores the ideal body fat percentage as an index for the purpose of dieting, separately for men and women. In this example, the target value acquisition section 57 estimates that the user's body composition values indicate that the user's gender-related physical characteristics are 70% male and 30% female.

The target value acquisition section 57 calculates the lower limit of the revised target value 571 by taking a weighted average (performing weighted addition) of the lower limit of the default target value 581 for men and the lower limit of the default target value 582 for women, based on the degree of male (70%) and female (30%) degrees. The lower limit of the revised target value 571 is calculated as a value obtained by proportionally dividing the lower limit of the default target value 581 for men and the lower limit of the default target value 582 for women in the ratio 30:70. Similarly, the upper limit is calculated by the target value acquisition section 57 by taking a weighted average (weighted addition) of the upper limit of the default target value for men 581 and the upper limit of the default target value for women 582, using the male (70%) and female (30%) degrees. The upper limit of the revised target value 571 is calculated as a value obtained by proportionally dividing the upper limit of the default target value 581 for men and the upper limit of the default target value 582 for women in the ratio 30:70.

The target value 571 calculated in this way is the ideal body fat percentage for a user with a male factor of 70% and a female factor of 30%, and this target value 571 is used as the target value for that user. The index value (in this case, body fat percentage (%),) 561 obtained by the index value acquisition section 56, indicated by the star symbol in Figure 4, is outside the default target value for women, assuming that the user's gender is female, but is within the range of the target value 571 that has been modified based on this user's physical characteristics, and is therefore evaluated as achieving an ideal body fat percentage.

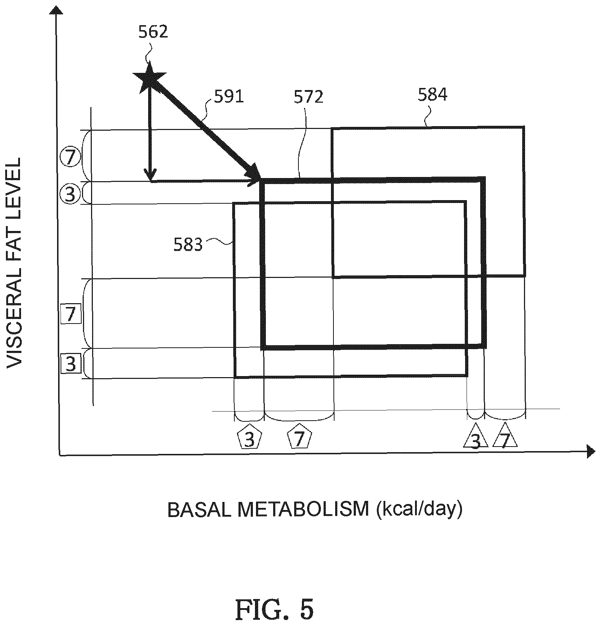

Figure 5 shows an example of modifying the target values for basal metabolism and visceral fat levels in an embodiment of this technology. The default target value memory section 58 stores the ideal combination of basal metabolism and visceral fat levels for each generation as an index for the purpose of anti-aging. In this example, the target value acquisition section 57 calculates that the internal age of a user is 38 years old based on the user's body composition.

The target value acquisition section 57 obtains the modified target value 572 for the lower limit of basal metabolism by taking a weighted average of the default target value 583 for those in their 30s (median age 35) and the default target value 584 for those in their 40s (median age 45), based on the estimated internal age of 38 years. The revised target value 572 for the lower limit of basal metabolism is calculated as a value obtained by proportionally dividing the lower limit of basal metabolism for the default target value 583 for the 30s and the lower limit of basal metabolism for the default target value 584 for the 40s in the ratio 30:70. Similarly, for the upper limit of basal metabolism, the target value acquisition section 57 calculates the upper limit of basal metabolism for the revised target value 572 by taking a weighted average of the upper limit of basal metabolism for the default target value 583 for those in their 30s and the upper limit of basal metabolism for those in their 40s, based on the estimated internal age of 38 years old. The modified target value 572 for the upper limit of basal metabolism is calculated as a value obtained by proportionally dividing the upper limit of basal metabolism for the default target value 583 for the 30s by 30:70, and the upper limit of basal metabolism for the default target value 584 for the 40s.

The target value acquisition section 57 obtains the lower limit of the target value 572 for visceral fat level in the same way as for the target value for visceral fat level, by taking a weighted average of the lower limit of the target value 583 for visceral fat level for the 30s and the lower limit of the target value 584 for visceral fat level for the 40s, based on the estimated internal age of 38 years. The revised target value 572 for the lower limit of visceral fat level is calculated as a value obtained by proportionally dividing the lower limit of visceral fat level for the default target value 583 for the 30s by 30 and the lower limit of visceral fat level for the default target value 584 for the 40s by 70. In addition, the target value acquisition section 57 calculates the upper limit of the revised target value 572 by taking a weighted average of the upper limit of the default target value 583 for the 30s and the upper limit of the visceral fat level for the 40s, based on the estimated body age of 38 years old. The upper limit of the revised target value 572 is calculated as a value obtained by proportionally dividing the upper limit of the default target value 583 for the 30s and the upper limit of the default target value 584 for the 40s by 30:70.

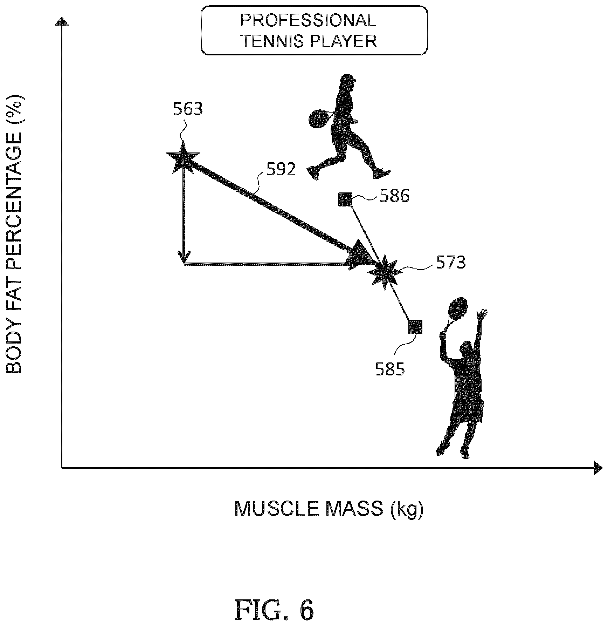

Figure 6 shows an example of modifying the target values for muscle mass and body fat percentage in an embodiment of this technology. The default target value memory section 58 stores, separately for men and women, the combination of muscle mass and body fat percentage as an index for the purpose of becoming a body like a professional tennis player. In this example, the target value acquisition section 57 estimates that a certain user's male degree is 60% and female degree is 40% based on that user's body composition.

The target value acquisition section 57 calculates the modified target value 573 muscle mass by taking a weighted average of the typical or average default target value 585 muscle mass of male professional tennis players and the typical or average default target value 586 muscle mass of female professional tennis players, weighted by the male (60%) and female (40%) degrees. The revised target muscle mass of 571 is calculated as a value obtained by proportionally dividing the default target muscle mass of 585 for male players and the default target muscle mass of 586 for female players in the ratio 40:60.

In addition, the target value acquisition section 57 calculates the modified target value 573 for body fat percentage by taking a weighted average of the default target value 585 for male players and the default target value 586 for female players, using the male (60%) and female (40%) degrees. The modified target value 571 for body fat percentage is calculated as a value obtained by proportionally dividing the default target value 585 for male players and the default target value 586 for female players in the ratio 40:60.

The screen information generation section 59 generates screen information that shows the relationship between the user's index value obtained by the index value acquisition section 56 and the target value for that user calculated by the target value acquisition section 57. The screen information generation section 59 may generate screens such as those shown in Figures 4 to 6. In the examples in Figures 4 to 6, the user's index values obtained by the indicator acquisition section 56 are indicated by a star symbol, i.e. body fat percentage 561 in the example in Figure 4, a combination of basal metabolism and visceral fat level 562 in the example in Figure 5, and a combination of muscle mass and body fat percentage 563 in the example in Figure 6.

Furthermore, in cases where the index is a combination of multiple items, as in Figures 5 and 6, the vectors 591 and 592 are shown, which point from the user's index value 562 and 563 to the modified target value 572 and 573. Furthermore, as shown in Figure 5, when the target value 572 is shown as a certain range defined by the upper and lower limits, the vector 591 from the user's index value 562 to the closest position within this range is shown. With these vectors 591 and 592, the user can visually understand how much they need to improve the body composition values of which items in order to achieve their target body.

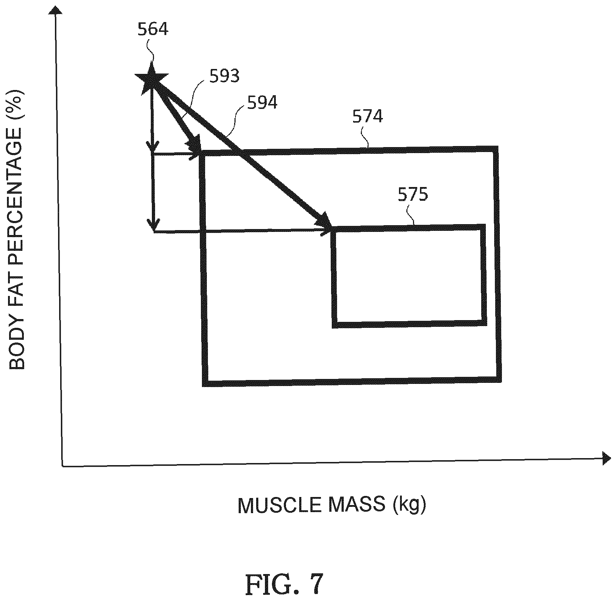

In the examples in Figures 4 to 6, the default target values used to modify the target values may be omitted. The following explains such examples. Figure 7 shows an example of the target values for muscle mass and body fat percentage in an embodiment of this technology. In the example in Figure 7, the target values for the combination of muscle mass and body fat percentage (564), the target value for the world ranking level of 1000 (574), and the target value for the world ranking level of 10 (575) are shown as indices for the purpose of aiming for the body of a tennis player. And the shortest vectors from the user's index values to the target values 574 and 575 are shown respectively as 593 and 594. In this way, it is possible to show the target values in multiple stages and show their relationship to the user's index value.

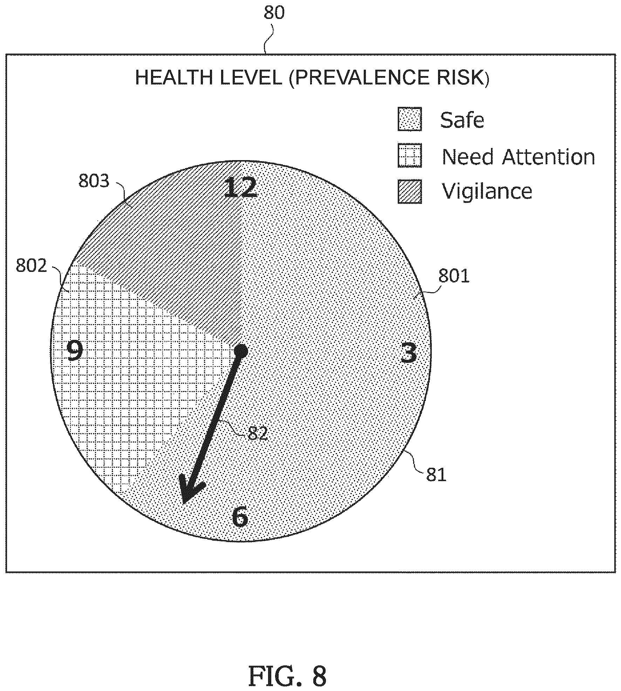

Figure 8 shows an example of an assessment result screen in an embodiment of this technology. In the example in Figure 8, the health level (prevalence risk) is shown as an index for the purpose of becoming healthier. In the example in Figure 8, the health level is determined as one of three values: healthy, attention required, or unhealthy. On screen 80, these are labeled as "Safe", "Need Attention", and "Vigilance", respectively. In this example, the target value is "Safe". The size of each of these "Safe", "Need Attention", and "Vigilance" regions is modified according to the user's physical characteristics, as described above.

On Screen 80, the health level (prevalence risk) is indicated by a clock-like face 81 and a hand 82 called a health meter. On the clock-like face 81, the sector from 0:00 to a specified time is the safe area 801, the sector from the safe area 801 to the specified time is the caution area 802, and the sector from the caution area 802 to 12:00 (0:00) is the vigilance area 803. In the example in Figure 8, the user's index value is shown by the hand 82, which is relatively close to "caution" and is "safe".

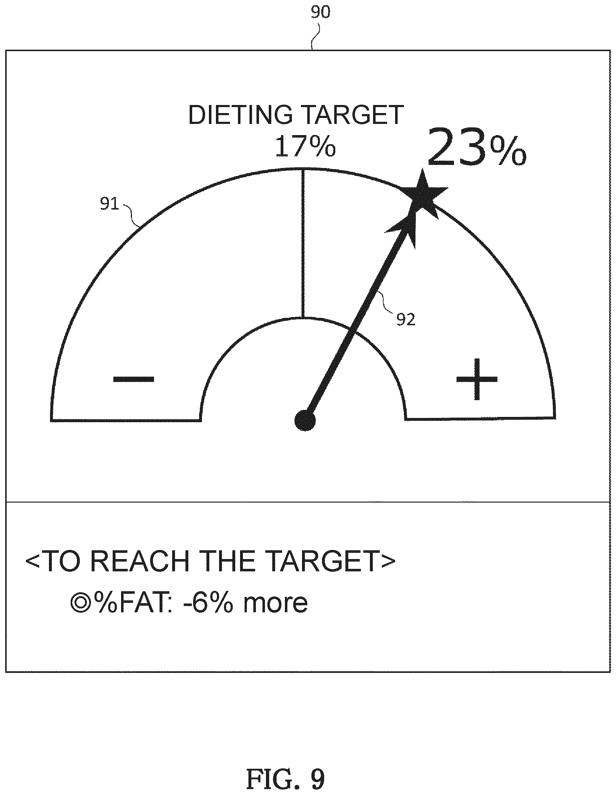

Figure 9 shows an example of an assessment result screen in an embodiment of this technology. In the example in Figure 9, the body fat percentage is shown as an index for the purpose of dieting. In the screen 90, the body fat percentage is shown with a tachometer-like scale 91 and needle 92. In this example, the target value is a body fat percentage of 17 , which is shown in the center of the scale 91. This target value is modified according to the user's physical characteristics, as described above.

In addition to the tachometer, the screen 90 also shows the body fat percentage that should be reduced or increased to reach the target value in text form. In the example in Figure 9, it is explained in text that the body fat percentage should be reduced by another 6%.

The output unit 60 outputs the screen information generated by the screen information generation unit 59. The output unit 60 may be the touch panel 11 of the information processing terminal 10, or the display panel 22 of the body composition analyzer 20. The output unit 60 may also be an external output module that transmits the screen information to another display device.

As described above, according to the body assessment system 100 of this embodiment, the target value that is compared with the index value obtained from the user's body composition value is calculated for each user according to the user's physical characteristics, so it is possible to set an appropriate target value and show the relationship with the user's index value without relying solely on the user's actual physical attributes.

In the above embodiment, the default target values corresponding to the physical attributes are stored in the default target value memory section 58, and by modifying these default target values, the target values corresponding to the user's physical characteristics are obtained. However, this is not the only method for obtaining target values corresponding to the user's physical characteristics. In order to obtain target values, it may be possible to obtain target values according to physical characteristics by selecting from target values prepared for each physical characteristic, rather than using the default target values established for each physical attribute. Alternatively, it may be possible to obtain target values for the relevant user by inputting the user's physical characteristics into an inference model in which physical characteristics are the input and target values are the output.

As described above, the body assessment system 100 of this embodiment comprises an information processing terminal 10 and a body composition analyzer 20, but the body composition estimation section 54, the index value acquisition section 56, the target value acquisition section 57, the default target value memory section 58 and the screen information generation 59 may be provided in either the information processing terminal 10 or the body composition scale 20, and the body assessment device shown in Figure 2 may be configured using only the body composition scale 20. Alternatively, the above elements may be implemented in the information processing terminal 10 by installing the body measurement program of this embodiment from a non-temporary storage medium. In addition, the body assessment system 100 may be composed of the body composition analyzer 20 and a server device connected via a communication network, and some or all of the above elements may be implemented in the server device.

[Reference Signs List]

    100 Body assessment system
    10 Information processing terminal
    11 Touch panel
    20 Body composition analyzer
    211R, 211L Current electrode
    212R, 212L Measurement electrode
    22 Display panel
    23 Operation buttons
    51 Height, age, and gender acquisition section
    52 Weight acquisition section
    53 BI acquisition section
    54 Body composition estimation section
    55 Purpose setting section
    56 Index value acquisition section
    57 Target value acquisition section
    58 Default target value memory section
    59 Screen information generation section
    60 Output section
    561-564 Index value
    571-575 Target value
    581-586 Default target value
    591-594 Vector
    80, 90 Screen

**Claims**

1. A body assessment system comprising:

   a body composition acquisition section that acquires body composition values for one or more items of a user, which are estimated based on the bioelectrical impedance of one or more body parts of the user;
   an index value acquisition section that acquires an index value of the user based on one or more body composition values for the index used to assess a body for a predetermined purpose;
   a target value acquisition section that calculates a target value for each of the users, according to the physical characteristics of the users, for the the index; and
   a screen information generation section that generates screen information showing a relationship between the index value and the target value.

2. The body assessment system according to claim 1, wherein the target value acquisition section may calculate the target value according to the predetermined purpose of the user.

3. The body assessment system according to claim 1, wherein the index value acquisition section selects one or more body composition items from the body composition values of the one or more items for assessing the body for the specified purpose, and acquires the body composition values of the selected items as the index values.

4. The body assessment system according to claim 1, wherein the index value acquisition section acquires the index value by inputting the body composition values of the one or more items into an inference model for calculating the index value.

5. The body assessment system according to any one of claim 1 to 4, wherein the target value acquisition section estimate the physical characteristics of the user based on the body composition values of one or more items acquired by the body composition acquisition section, and calculates the target value based on the estimated physical characteristics.

6. The body assessment system according to claim 5, further comprising a default target value memory that stores default target values for each physical attribute for the index, wherein the target value acquisition section calculates the target value based on the estimated physical characteristics and the default target values.

7. The body assessment system according to claim 6, wherein the default target value memory stores the target values according to the predetermined purpose.

8. The body assessment system according to claim 6, wherein the target value acquisition section estimates the degree of the physical attributes of the user as the physical characteristics, and calculates the target value by weighting and adding the default target values for each of the physical attributes according to the degree of the physical attributes.

9. The body assessment system according to claim 8, wherein

   the physical attribute is gender,
   the default target value memory section stores default target values for males and females respectively, and
   the target value acquisition section calculates the target value by estimating a gender degree of the user as the degree of the physical attribute of the user based on the body composition values of one or more items, and calculates the targe value by weighting and adding the default target values for males and females according to the estimated gender degree.

10. The body assessment system according to claim 8, wherein

    the physical attribute is age,
    the default target value acquisition section stores the default target values for each generation according to the purpose, and
    the target value acquisition section calculates the target value by estimating an internal age of the user as the degree of the physical attributes of the user based on the body composition values of one or more items, and calculates the target value by weighting and adding the default target values for each generation according to the estimated internal age.

11. The body assessment system according to claim 8, wherein

    the physical attribute is race,
    the default target value acquisition section stores the default target values for each race, and
    the target value acquisition section calculates the target value by estimating a racial degree of the user as the degree of the physical attribute of the user based on the body composition values of the one or more items, and calculates the target value by weighting and adding the default target values for each race according to the estimated racial degree.

12. The body assessment system according to any one of claims 1 to 11, wherein the target value acquisition section calculates the target value as a target range defined by an upper limit and a lower limit.

13. The body assessment system according to any one of claims 1 to 12, wherein the purpose is selectable from at least one of health, dieting, anti-aging, ideal athletes for a specific sport, and specific celebrities.

14. The body assessment system according to any one of claims 1 to 10, further comprising:

a bioelectrical impedance acquisition section that acquires the bioelectrical impedance of one or more body parts of the user by measuring the user's body; and
a estimation section that estimates the body composition based on the bioelectrical impedance, wherein
the body composition acquisition section acquires the body composition value of the user estimated by the estimation section based on the bioelectrical impedance acquired by the bioelectrical impedance acquisition section.

15. A body assessment program configured so that when executed by a computer, the computer functions as:

a body composition acquisition section that acquires body composition values for one or more items of a user, which are estimated based on the bioelectrical impedance of one or more body parts of the user;
an index value acquisition section that acquires an index value of the user based on one or more body composition values for the index used to assess a body for a predetermined purpose;
a target value acquisition section that calculates a target value for each of the users, according to the physical characteristics of the users, for the the index; and
a screen information generation section that generates screen information showing a relationship between the index value and the target value.

FIG. 1

FIG. 2

FIG. 3

FIG. 4

EP 4 461 215 A1

BASAL METABOLISM (kcal/day)

FIG. 5

FIG. 6

FIG. 7

FIG. 8

FIG. 9

# EP 4 461 215 A1

**INTERNATIONAL SEARCH REPORT**

| International application No. |
|---|
| **PCT/JP2023/000024** |

**A. CLASSIFICATION OF SUBJECT MATTER**

*A61B 5/0537*(2021.01)i; *G16H 20/00*(2018.01)i
FI: A61B5/0537 100; G16H20/00

According to International Patent Classification (IPC) or to both national classification and IPC

**B. FIELDS SEARCHED**

Minimum documentation searched (classification system followed by classification symbols)

A61B5/053-5/0537; G06Q50/22; G16H10/00-80/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-194198 A (TANITA CORP.) 03 December 2020 (2020-12-03) paragraphs [0005], [0006], [0020], [0021], [0023]-[0028], [0030]-[0035], [0039], [0044]-[0047], [0049], [0053], fig. 2-4, 8 | 1-4, 12-15 |
| A | JP 2004-337578 A (YAMATO SCALE CO., LTD.) 02 December 2004 (2004-12-02) paragraphs [0026]-[0077], fig. 1-6 | 1-15 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/000024**

| Patent document cited in search report | | | Publication date (day/month/year) | Patent family member(s) | Publication date (day/month/year) |
|---|---|---|---|---|---|
| JP | 2020-194198 | A | 03 December 2020 | (Family: none) | |
| JP | 2004-337578 | A | 02 December 2004 | JP 2010-57953 A paragraphs [0028]-[0081], fig. 1-6 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022000697 A

- JP 2002063278 A